# EUROPEAN PATENT SPECIFICATION

(11) **EP 1 640 032 B1**
(45) Date of publication and mention of the grant of the patent: **20.02.2008**
(21) Application number: 05020458.5
(22) Date of filing: 20.09.2005
(51) Int. Cl.: A61M 15/08, A61M 15/00

(54) **Medicine administering device for nasal cavities**
Vorrichtung zum Verabreichen eines Medikamentes in die Nasenhöhlen
Appareil pour administrer des médicaments dans les cavités nasales

(30) Priority: 27.09.2004 JP 2004279629
(43) Date of publication of application: 29.03.2006
(73) Proprietor: HITACHI, LTD., Chiyoda-ku Tokyo 100-8280 (JP); DOTT LIMITED COMPANY, Yokohama-shi, Kanagawa 224-0051 (JP)
(72) Inventor: Ishizeki, Kazunori Automotive Systems, HITACHI LTD, Isesaki-shi Gumma 372-0023 (JP); Nakamura, Shigemi Automotive Systems, HITACHI LTD, Isesaki-shi Gumma 372-0023 (JP); Ohki, Hisatomo Automotive Systems, HITACHI LTD, Isesaki-shi Gumma 372-0023 (JP); Yanagawa, Akira DOTT LIMITED COMPANY, Yokohama-shi Kanagawa 224-0024 (JP)
(74) Representative: Schaeberle, Steffen

(56) References cited:
- EP-A- 0 779 078
- DE-A1- 4 021 263
- US-A1- 2002 073 991
- US-A1- 2002 158 150

## Description

### BACKGROUND OF THE INVENTION

The present invention relates to improvements in a medicine administering device suitable for administering powdery medicine into nasal cavities of a patient according to the preamble part of claim 1. Such a device is known from DE-A-40 21 263.

In general, to cure patients suffering from nasal allergy or the like, curing methods in which powdery medicine is administered through nasal cavities of the patients have been employed. Hitherto, various medicine administering devices for nasal cavities have been proposed

Japanese Patent Examined Publication 63-6024 discloses a medicine administering device for nasal cavities, having a medicine administering device main body; a spray nozzle that is fixed to a tip end side of the main body and is to be inserted into the nasal cavities; and a rubber pump member attached to a base end side of the main body.

In order to administer a powdery medicine into the nasal cavities by using the above conventional medicine administering device, first the spray nozzle is inserted into one of the left and right nasal cavities. Then, in this state, the pump member is pressed to compressively deform a pump chamber. With this, the air of the pump chamber is supplied into a medicine accommodating chamber to pressurize the medicine accommodating chamber. Thus, a powdery medicine is transported through a medicine transporting passage of the medicine administering device and then sprayed together with the air from the spray nozzle into the nasal cavities.

In the case of the above conventional medicine administering device, it is necessary in each administration to charge the medicine accommodating chamber with a powdery medicine in the form of capsule. This is a cumbersome procedure.

### SUMMARY OF THE INVENTION

It is therefore an object of the present invention to provide a medicine administering device for nasal cavities, which can reduce a burden of charging the device with a powdery medicine.

To solve this object, according to the present invention, there is provided a medicine administering device for nasal cavities according to claim 1. The subclaims contain preferred embodiments of the invention.

The other objects and features of this invention will become understood from the following description with reference to the accompanying drawings.

### BRIEF DESCRIPTION OF THE DRAWINGS

Fig. 1 is a frontal view showing a medicine administering device for nasal cavities according to an embodiment of the present invention;
Fig. 2 is a sectional view taken along the lines II-II of Fig. 1;
Fig. 3 is an enlarged frontal view showing a medicine accommodating magazine of Fig. 1;
Fig. 4 is an enlarged plan view showing the medicine accommodating magazine;
Fig. 5 is a sectional view taken along the lines V-V of Fig. 4; and
Fig. 6 is an enlarged sectional view showing spray nozzle portions of the medicine administering device.

### DESCRIPTION OF THE PREFERRED EMBODIMENTS

As stated above, a medicine administering device for nasal cavities according to the present invention has a medicine accommodating chamber configured to accommodate at once a powdery medicine used in a plurality of administrations. With this, it is not necessary in each administration to charge the medicine accommodating chamber with a powdery medicine. Therefore, it is possible by the present invention to reduce a burden of charging the device with a powdery medicine and to efficiently conduct a medicine administration.

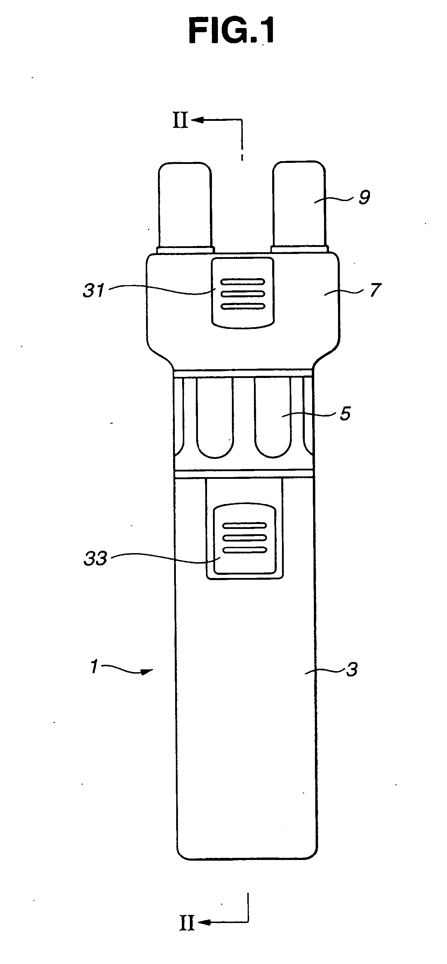

With reference to Figs. 1-6, a medicine administering device for nasal cavities according to an embodiment of the present invention is described in detail in the following.

As is seen from Fig. 1, medicine administering device for nasal cavities 1 is equipped with pump 3, medicine accommodating chamber (magazine) 5 disposed above pump 3, holding member 7 disposed above medicine accommodating magazine 5, and a pair of spray nozzle portions 9, 9 that are held by and project from holding member 7.

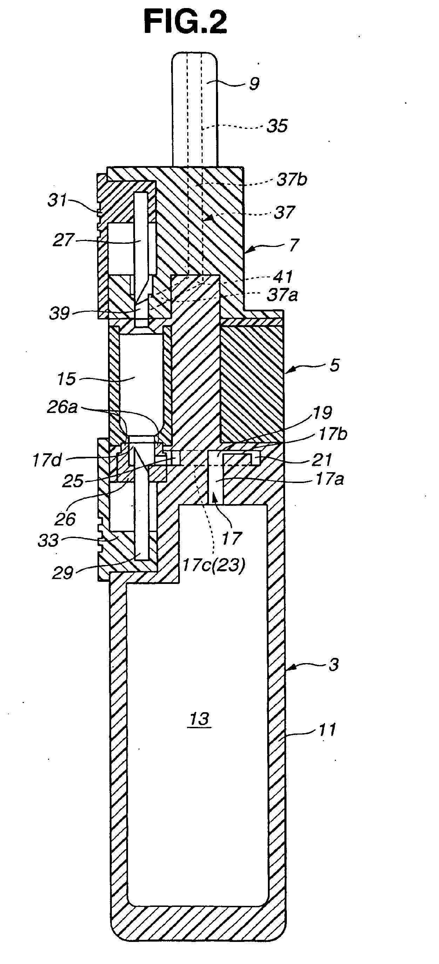

As is seen from Fig. 2, pump 3 is formed of pump member 11 that is cylindrical in shape and is made of rubber. Pump chamber 13 is formed in the inside of pump member 11.

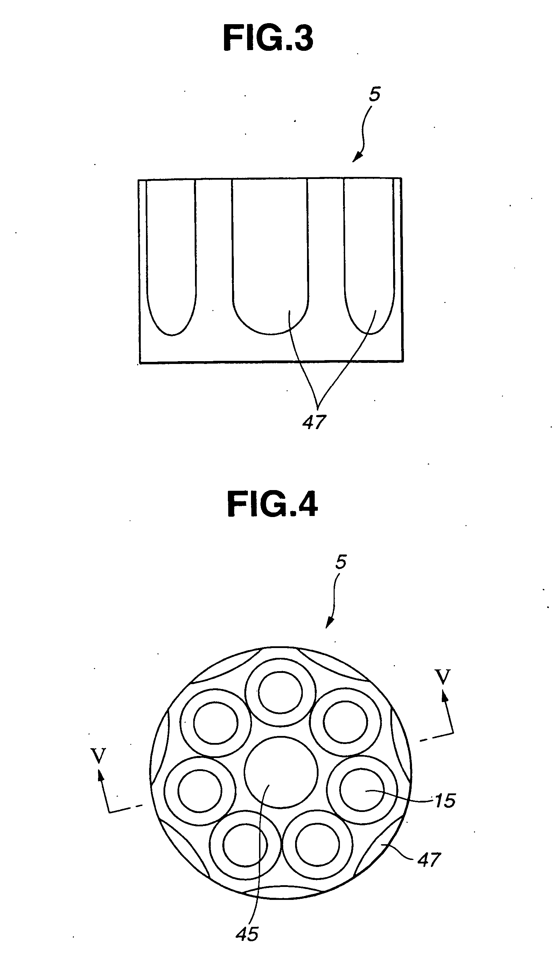

As is seen from Fig. 4, a plurality (seven in Fig. 4) of medicine accommodating compartments 15 are formed in the inside of medicine accommodating magazine 5. As shown in Fig 2, medicine accommodating compartment 15 in position for administration communicates with pump chamber 13 through first air passage 17.

First air passage 17 is formed of straight passage portion 17a upwardly extending from pump chamber 13, first communication passage portion 17b horizontally extending from the top of straight passage portion 17a, curved passage portion 17c extending in a semicircular manner in the circumferential direction of medicine administering device 1 from a terminal end of communication passage portion 17b, and second communication passage portion 17d extending between a terminal end of curved passage portion 17c and medicine accommodating compartment 15 in position for administration.

Perpendicularly bent portions 19 and 21 are respectively provided between straight passage portion 17a and first communication passage portion 17b and between first communication passage portion 17b and curved passage portion 17c. The semicircular passage of the curved passage portion 17c itself is provided with substantially perpendicularly bent portion 23. Substantially perpendicularly bent portion 25 is provided between the terminal end of the curved passage portion 17c and second communication passage portion 17d.

By the provision of bent portions 19, 21, 23 and 25, it is possible to suitably control flow rate and flow velocity of the air supplied to medicine accommodating compartment 15 and to adjust the degree of diffusion of medicine depending on the type of medicine. Thus, it is possible to conduct a stable medicine administration.

Medicine accommodating compartment 15 has a substantially cylindrical form for accommodating therein a capsule (not shown in the drawings) charged with a powdery medicine. Above and below medicine accommodating compartment 15 there are respectively disposed first and second perforators 27 and 29 for perforating or opening the capsule in position for administration.

As shown in Figs. 1 and 2, first perforator 27 is fixed to first slider 31 and thus is allowed to slide down by moving first slider 31 down to perforate the top of the capsule. Second perforator 29 is fixed to second slider 33 and thus is allowed to slide up by moving second slider 33 up to perforate the bottom of the capsule.

Medicine accommodating compartment 15 in position communicates with medicine passage 35 of each spray nozzle portion 9 through second air passage 37. Second air passage 37 is formed of (a) first passage portion 37a that extends obliquely upwardly from medicine accommodating compartment 15 and (b) second passage portion 37b that is in line with medicine passage 35. First passage portion 37a is formed at its first and second ends with bent portions 39 and 41, respectively. By the provision of bent portions 39 and 41, it is possible to efficiently prevent reverse flow of the powdery medicine from medicine passages 35 of spray nozzle portions 9, 9. Thus, bent portions 39 and 41 can act as a temporary medicine trap.

As is seen from Fig. 2, medicine receiver 26 for receiving the powdery medicine that drops from medicine accommodating chamber 15 is formed under medicine accommodating chamber 15 in position. This medicine receiver 26 has a cylindrical base portion and annular projection 26a formed on the cylindrical base portion. Medicine receiver 26 also serves as a bush or collar for supporting second perforator 29. In fact, medicine receiver 26 has a lower cylindrical void space for supporting the sliding movement of second perforator 29 and an upper cylindrical void space (formed on the lower cylindrical void space) for receiving the powdery medicine that drops from medicine accommodating chamber 15. Annular projection 26a can be brought into a temporary engagement in a snap action manner with a bottom notch formed below each medicine accommodating chamber 15, by rotating medicine accommodating magazine 5. Thus, each medicine accommodating chamber 15 can be stopped at proper position for administration shown in Fig. 2 by this temporary engagement. Annular projection 26a can have a convex top surface to smoothly provide the temporary engagement and disengagement in alternation by rotating medicine accommodating magazine 5. Medicine accommodating magazine 5 has seven bottom notches at respective positions below seven medicine accommodating chambers 15. Furthermore, medicine receiver 26 has a passage portion for diffusing the air supplied from pump 3. This passage portion extends outwardly and horizontally from the bottom of the upper void space of medicine receiver 26. The diffused air can efficiently force the powdery medicine (including the powdery medicine dropped from medicine accommodating chamber 15) to move toward spray nozzle portions 9, 9.

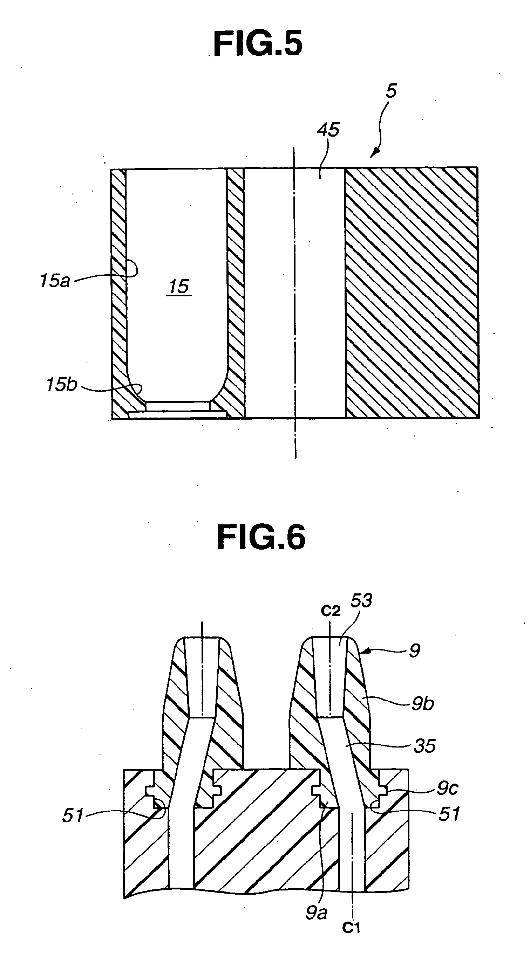

As shown in Figs. 3-5, medicine accommodating magazine 5 is substantially cylindrical in form and is formed at its central portion in diametral direction with supporting through hole 45. Furthermore, medicine accommodating magazine 5 is formed on its outside surface with a plurality (seven in Fig. 4) of gripping notches 47 that are arranged at regular intervals in circumferential direction. Each gripping notch 47 is a depression formed inward in radial direction and has a shape suitable for gripping by fingertips of a patient. As is shown in Fig. 5, each medicine accommodating compartment 15 extends along the axial direction of the medicine accommodating magazine 5.

Each medicine accommodating compartment 15 has upper side surface 15a that is cylindrical in shape and bottom surface 15b that is inwardly curved. As shown in Fig. 4, seven of medicine accommodating compartments 15 are arranged at regular intervals in circumferential direction.

As is seen from Fig. 6, each spray nozzle portion 9 is rotatably fit into nozzle holding hole 51 formed in holding member 7. Each spray nozzle portion 9 is a monolithic member and has base portion 9a that is inserted into nozzle holding hole 51 and main body 9b that is positioned above base portion 9a. Nozzle main body 9b is formed into a substantially cylindrical shape having a diameter and an axial length, which are suitable for insertion into a nasal cavity of a patient. Center axis C2 of nozzle main body 9b is designed to be away from center axis (rotation center) C1 of base portion 9a of spray nozzle portion 9. Therefore, it becomes possible to change the distance between nozzle main bodies 9b, 9b by rotating the spray nozzle portions 9, 9. In other words, it is possible to adjust the distance between spray openings 53, 53 depending on the distance of nasal cavities of a patient.

Each base portion 9a of spray nozzle portion 9 is formed at its outer surface with a projection that extends circumferentially and fit into an annular groove formed in holding member 7. With this, each spray nozzle portion 9 is rotatably held in position. Furthermore, each spray nozzle portion 9 is formed with medicine passage 35 that communicates at its lower end opening with second air passage 37. This lower end opening of medicine passage 35 has center axis C1 at its center. Medicine passage 35 is formed at its upper end with spray opening 53 having center axis C2 at its center. Thus, medicine passage 35 is formed into an oblique passage extending between second air passage 37 and spray opening 53.

An exemplary medicine administration using the medicine administering device for nasal cavities is explained in the following.

At first, medicine accommodating magazine 5 is charged with at least one unused capsule enclosing therein a powdery medicine. In case that the distance between spray nozzle portions 9, 9 does not fit to the distance between nasal cavities of a patient, the former is adjusted by rotating the spray nozzle portions 9, 9 until spray nozzle portions 9, 9 fit to the nasal cavities. Then, while gripping notches 47 of medicine accommodating magazine 5 are gripped by the patient's fingers, medicine accommodating magazine 5 is rotated by a patient until an unused capsule comes to a position where the capsule is opposed to or inserted between first and second perforators 27 and 29. Then, first and second sliders 31 and 33 are respectively slid downwardly and upwardly to perforate the capsule by first and second perforators 27 and 29.

Then, nozzle main bodies 9b, 9b are inserted into the right and left nasal cavities of a patient. Under this state, pump member 11 is pressed to compressively deform pump chamber 13. With this, air is introduced into medicine accommodating compartment 15 in position shown in Fig. 2 through first air passage 17 to pressurize this medicine accommodating compartment 15. Thus, the powdery medicine of medicine accommodating compartment 15 is forced to upwardly move by the air through air passages 37, 37 and then medicine passages 35, 35. Then, the powdery medicine is sprayed from the spray openings 53, 53 into the right and left nasal cavities.

Although the invention has been described above by reference to certain embodiments of the invention, the invention is not limited to the embodiments described above. Modifications and variations of the embodiments described above will occur to those skilled in the art, in light of the above teachings. The scope of the invention is defined with reference to the following claims.

## Claims

1. A medicine administering device (1) for nasal cavities, comprising:
a medicine accommodating magazine (5) configured to accommodate at once a powdery medicine used in a plurality of administrations, the medicine accommodating magazine (5) having a substantially cylindrical form and being held by a base portion of the medicine administering device (1) to be rotatable in a circumferential direction of the medicine accommodating magazine (5), the medicine accommodating magazine (5) having a plurality of medicine accommodating compartments (15) that are arranged in the circumferential direction, wherein the medicine accommodating magazine (5) has a portion defining each medicine accommodating compartment (15) in the medicine accommodating magazine (5);
a spray nozzle (9) for spraying the powdery medicine into nasal cavities, the spray nozzle (9) communicating with the medicine accommodating magazine (5); and
a pump (3) for supplying air into the medicine accommodating magazine (5) and forcing the powdery medicine by the air out of the spray nozzle (9) into the nasal cavities, being **characterized in**
**that** a medicine receiver (26) for receiving the powdery medicine that drops from the medicine accommodating magazine (5) is formed between the medicine accommodating magazine (5) and the pump (3),
**that** the medicine receiver (26) is formed with a projection portion (26a) that is engaged with the portion of the medicine accommodating magazine (5), and
**that** the medicine receiver (26) is formed with a passage portion for diffusing the air from the pump (3).

2. A medicine administering device (1) according to claim 1, wherein the spray nozzle (9) comprises a pair of spray nozzle portions (9, 9) to be inserted into the nasal cavities, the spray nozzle portions (9) being configured such that a distance between openings (53, 53) of the spray nozzle portions (9, 9) is adjustable.

3. A medicine administering device (1) according to claim 2, wherein the spray nozzle portions (9, 9) are rotatably fixed to a base portion (7) of the medicine administering device (1), each spray nozzle portion (9) being formed at a center of the opening (53) of each spray nozzle portion (9) with a center line (C2) that is away from a rotation center (C1) of each spray nozzle portion (9) such that the distance between the openings (53, 53) of the spray nozzle portions (9, 9) is adjustable by rotating at least one of the spray nozzle portions (9, 9).

4. A medicine administering device (1) according to any one of claims 1-3, wherein an air passage (17, 37) for supplying the air from the pump (3) to the spray nozzle (9) is formed between the pump (3) and the spray nozzle (9), the air passage (17, 37) having a bent portion (19, 21, 23, 25, 39, 41).

## Patentansprüche

1. Arzneimittel-Applizierungsvorrichtung (1) für Nasenhöhlen, umfassend:
ein Arzneimittel-Aufnahmemagazin (5), das ausgelegt ist, auf einmal ein pulverförmiges Arzneimittel aufzunehmen, das für eine Vielzahl von Applizierungen verwendet wird, wobei das Arzneimittel-Aufnahmemagazin (5) eine im wesentlichen zylindrische Gestalt aufweist und durch einen Basisbereich der Arzneimittel-Applizierungsvorrichtung (1) so gehalten wird, dass es in einer Umfangsrichtung des Arzneimittel-Aufnahmemagazins (5) rotierbar ist, wobei das Arzneimittel-Aufnahmemagazin (5) eine Vielzahl von Arzneimittel-Aufnahmekammern (15) aufweist, die in der Umfangsrichtung angeordnet sind, wobei das Arzneimittel-Aufnahmemagazin (5) einen Bereich aufweist, der jede Arzneimittel-Aufnahmekammer (15) in dem Arzneimittel-Aufnahmemagazin (5) begrenzt;
eine Sprühdüse (9) zum Versprühen des pulverförmigen Arzneimittels in Nasenhöhlen, wobei die Sprühdüse (9) mit dem Arzneimittel-Aufnahmemagazin (5) kommuniziert; und
eine Pumpe (3) um in das Arzneimittel-Aufnahmemagazin (5) Luft einzuleiten und das pulverförmige Arzneimittel durch die Luft aus der Sprühdüse (9) in die Nasenhöhlen zu treiben, **dadurch gekennzeichnet,**
**dass** eine Arzneimittel-Aufnahmeeinrichtung (26) zum Aufnehmen des pulverförmigen Arzneimittels, das aus dem Arzneimittel-Aufnahmemagazin (5) herausfällt, zwischen dem Arzneimittel-Aufnahmemagazin (5) und der Pumpe (3) ausgebildet ist,
**dass** die Arzneimittel-Aufnahmeeinrichtung (26) mit einem hervorstehenden Bereich (26a) ausgebildet ist, der mit dem Bereich des Arzneimittel-Aufnahmemagazins (5) im Eingriff ist, und
**dass** die Arzneimittel-Aufnahmeeinrichtung (26) mit einem Durchgangsbereich ausgebildet ist, um die Luft von der Pumpe (3) zu verströmen.

2. Arzneimittel-Applizierungsvorrichtung (1) gemäß Anspruch 1, wobei die Sprühdüse (9) ein Paar von Sprühdüsenbereichen (9, 9) umfasst, das in die Nasenhöhlen einzuführen ist, wobei die Sprühdüsenbereiche (9) so ausgelegt sind, dass ein Abstand zwischen Öffnungen (53, 53) der Sprühdüsenbereiche (9, 9) einstellbar ist.

3. Arzneimittel-Applikationsvorrichtung (1) gemäß Anspruch 2, wobei die Sprühdüsenbereiche (9, 9) rotierbar an einem Basisbereich (7) der Arzneimittel-Applizierungsvorrichtung (1) angebracht sind, wobei jeder Sprühdüsenbereich (9) an einem Zentrum der Öffnung (53) von jedem Sprühdüsenbereich (9) mit einer Mittelachse (C2) ausgebildet ist, die derart von einem Rotationszentrum (C1) jedes Sprühdüsenbereichs (9) entfernt ist, dass der Abstand zwischen den Öffnungen (53, 53) der Sprühdüsenbereiche (9, 9) durch das Rotieren mindestens eines der Sprühdüsenbereiche (9, 9) einstellbar ist.

4. Arzneimittel-Applizierungsvorrichtung (1) gemäß einem der Ansprüche 1-3, wobei ein Luftdurchgang (17, 37) zum Zuführen der Luft von der Pumpe (3) zu der Sprühdüse (9) zwischen der Pumpe (3) und der Sprühdüse (9) ausgebildet ist, wobei der Luftdurchgang (17, 37) einen gekrümmten Bereich (19, 21, 23, 25, 39, 41) aufweist.

## Revendications

1. Dispositif d'administration d'un médicament (1) pour les cavités nasales, comprenant :
un caisson de logement de médicament (5) configuré pour loger au moins un médicament en poudre utilisé dans une pluralité d'administrations, le caisson de logement de médicament (5) présentant une forme sensiblement cylindrique et étant maintenu par une partie de base du dispositif d'administration de médicament (1) pour pouvoir être amené en rotation dans une direction circonférentielle du caisson de logement de médicament (5), le caisson de logement de médicament (5) présentant une pluralité de compartiments de logement de médicament (15) qui sont agencés dans la direction circonférentielle, dans lequel le caisson de logement de médicament (5) présente une partie définissant chaque compartiment de logement de médicament (15) dans le caisson de logement de médicament (5) ;
une buse de pulvérisation (9) destinée à pulvériser le médicament en poudre à l'intérieur des cavités nasales, la buse de pulvérisation (9) communiquant avec le caisson de logement de médicament (5) ; et
une pompe (3) destinée à amener de l'air à l'intérieur du caisson de logement de médicament (5) et poussant le médicament en poudre par l'air à l'extérieur de la buse de pulvérisation (9) à l'intérieur des cavités nasales,
**caractérisé en ce que**
un récepteur de médicament (26) destiné à recevoir le médicament en poudre qui tombe du caisson de logement de médicament (5) est formé entre le caisson de logement de médicament (5) et la pompe (3),
le récepteur de médicament (26) est formé avec une partie en saillie (26a) qui est mise en prise avec la partie du caisson de logement de médicament (5) ; et
le récepteur de médicament (26) est formé avec une partie de passage destinée à diffuser l'air provenant de la pompe (3).

2. Dispositif d'administration de médicament (1) selon la revendication 1, dans lequel la buse de pulvérisation (9) comprend une paire de parties de buse de pulvérisation (9, 9) à insérer à l'intérieur des cavités nasales, les parties de buse de pulvérisation (9) étant configurées de telle sorte qu'une distance entre les ouvertures (53, 53) des parties de buse de pulvérisation (9, 9) est réglable.

3. Dispositif d'administration de médicament (1) selon la revendication 2, dans lequel les parties de buse de pulvérisation (9, 9) sont fixées de manière rotative à une partie de base (7) du dispositif d'administration de médicament (1), chaque partie de buse de pulvérisation (9) étant formée au niveau d'un centre de l'ouverture (53) de chaque partie de buse de pulvérisation (9) avec une ligne médiane (C2) qui est distante d'un centre de rotation (C1) de chaque partie de buse de pulvérisation (9) de telle sorte que la distance entre les ouvertures (53, 53) des parties de buse de pulvérisation (9, 9) peut être réglée en amenant en rotation au moins une des parties de buse de pulvérisation (9, 9).

4. Dispositif d'administration de médicament (1) selon l'une quelconque des revendications 1 à 3, dans lequel un passage d'air (17, 37) destiné à amener l'air provenant de la pompe (3) à la buse de pulvérisation (9) est formé entre la pompe (3) et la buse de pulvérisation (9), le passage d'air (17, 37) présentant une partie coudée (19, 21, 23, 25, 39, 41).
